Europäisches Patentamt

European Patent Office

Office européen des brevets

(11) Publication number: **0 191 646**
**B1**

## (12) EUROPEAN PATENT SPECIFICATION

(45) Date of publication of patent specification: **02.05.90**

(51) Int. Cl.⁵: **A 61 F 5/44**

(21) Application number: **86301034.4**

(22) Date of filing: **14.02.86**

(54) Gas filter.

(30) Priority: **14.02.85 GB 8503749**
**16.07.85 GB 8517872**

(43) Date of publication of application:
**20.08.86 Bulletin 86/34**

(45) Publication of the grant of the patent:
**02.05.90 Bulletin 90/18**

(84) Designated Contracting States:
**AT BE CH DE FR IT LI LU NL SE**

(56) References cited:
**FR-A-2 357 291**
**GB-A-2 059 797**
**GB-A-2 114 444**
**GB-A-2 116 433**
**US-A-3 952 727**

(73) Proprietor: **E.R. Squibb & Sons, Inc.**
**Lawrenceville-Princeton Road**
**Princeton, N.J. 08540-4000 (US)**

(72) Inventor: **Steer, Peter Leslie**
**Woodlands Rise, Kingscote**
**East Grinstead, Sussex (GB)**

(74) Representative: **Cook, Anthony John et al**
**D. YOUNG & CO. 10, Staple Inn**
**London, WC1V 7RD (GB)**

Courier Press, Leamington Spa, England.

**Description**

In particular, the invention relates to a gas filter primarily intended for use in or with an ostomy appliance.

An ostomy appliance, sometimes called an ostomy bag, is a collection bag for receiving drainage from an abdominal opening following surgery, and an ostomy appliance is normally considered to comprise such a bag and means whereby it can be attached to a wearer.

Ostomy bags are well known. Since the discharge of the wearer of the bag often contains flatus gases, there have been continuing research and development efforts to device a satisfactory way of allowing such gases to escape from the interior of the bag in such a way that they are filtered and deodorized. Examples of prior art ostomy bag filter arrangements are seen in U.S. Patent No. A—3 952 727 Nolan *et al*, Patent No. GB—A—1 596 496, French Patent Specification, No. A—2357291, and Patent No. GB—A—2 059 797. These documents suggest matted fibres carrying activated carbon or carbon cloth as the filter medium. U.K. Application GB—A—2 116 433 makes a similar suggestion but mentions in passing that the filter may be of open-celled plastics foam. U.S. Patent No. A—4 427 425 Briggs *et al* makes another suggestion to use carbon cloth.

Known gas filters for ostomy bags, which are sometimes also called ostomy pouches, include gas filters which are attached to ostomy bags during manufacture as well as gas filters which can be attached to bags which were not specifically designed to have gas filters. Such prior designs did not yield a product which could be worn safely when swimming or when taking a shower. In other words, prior designs of ostomy bag filters were not waterproof to the extent desirable. In addition, previously known ostomy bag filters failed to allow passage of an adequate gas flow after being immersed in water.

Accordingly, a gas filter which could be attached to an ostomy bag which was not specially designed for such a filter and which would continue to operate, even after being wetted, would be desirable.

According to the present invention, there is provided a gas filter for an ostomy bag comprising a pad impregnated with activated carbon as a deodorizing agent and made of an open cell plastic foam material which is laminated together with a first microporous membrane, there being a layer of water repellent material on the surface of the membrane other than that to which said pad is attached, said layer being sufficiently thin that the pores in the membrane are not blocked against gas flow, characterised in that the pad is reticulated polyurethane foam having the characteristics indicated in the following Table A:

TABLE A

| Density | 27—30 kg/m$^3$ |
|---|---|
| Compression resistance | 3.4—4.6 KPa |
| Ultimate elongation | 350% |
| Tensile strength | 200 KPa |
| Tear strength | 6 N/cm |
| Porosity | 65—85 (PPI); |

and further characterised in that the pad carries substantially 180 to 220 grams of carbon per square meter, and having a thickness in the manufactured state of the filter of approximately 1 millimetre.

A filter carrier and a filter for ostomy use are disclosed herein. The filter includes a pad of reticulated polyurethane foam carrying particles of a deodorizing agent, and the filter carrier has a plastic housing which has a base wall having a hole formed therethrough which allows entry of gas to be deodorized and a retaining cap which is attached to the base wall so as to contain the foam pad within the housing. The peripheral wall is constructed to prevent the escape of any gas which exits from the edges of the pad. A water repellent coating is applied on at least the exposed surface of the pad within the peripheral wall.

In the drawing
   Figure 1 is a side view of the present invention mounted on an ostomy bag;
   Figure 2 is a cross-sectional view of the gas filter and the filter housing of Figure 1;
   Figure 3 is a top view of the filter housing of Figure 1 without the filter; and
   Figure 4 is a bottom view of the filter housing of Figure 1.

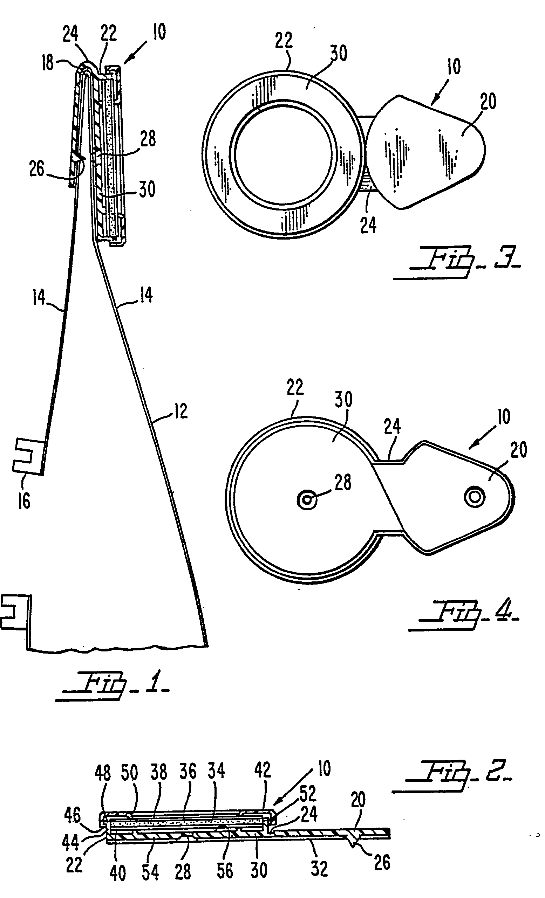

Referring generally to Figure 1, the present invention is a gas filter 10 which is intended for use in connection with an ostomy bag 12 of a type which is well known in the art. The ostomy bag 12 is typically comprised of a bag having plastic film walls 14 and a sealing connector 16 which is adapted to be attached to a mating connector (not shown) which is adhesively attached to a wearer.

In use, the ostomy bag 12 collects material and gases. The purpose of the gas filter 10 of the present invention is to provide a means for venting gases from the ostomy bag 12 while deodorizing the gases which are vented. The gas filter 10 is adapted to be attached like a cuff link to an ostomy bag 12 which has not been specially adapted to receive a gas filter. Accordingly, the present invention provides a means for both attaching to the ostomy bag 12 and for providing a vent through the ostomy bag 12.

In accordance with the invention, the gas filter 10 attaches to the ostomy bag 12 by clipping over an edge 18 of the ostomy bag 12. A flap 20 on the gas filter 10 is attached to the filter housing 22 by means of a hinge 24. A spike 26 on the flap 20 is used to puncture a hole through both walls 14 of the ostomy bag 12. The spike 26 is aligned with an opening 28 on the base wall 30 of the filter housing 22. Accordingly, when the gas filter 10 is connected to the ostomy bag 12 the spike 26 serves to provide an opening through the ostomy bag 12 which is aligned with the opening 28 in the base wall 30 of the filter housing 22. The base wall 30 of the filter housing 22 and the surface of the flap 20 are each covered with an adhesive layer 32 whose purpose is to hold the gas filter 10 in place on the ostomy bag 12.

Referring now to Figures 2—4, the construction of the gas filter 10 is described. The gas filter 10 includes a filter housing 22, which is substantially cylindrical in the preferred embodiment of the invention. The filter housing 22 holds a filter 34 which is comprised of a reticulated polyurethane foam pad 36 which is sandwiched between a first membrane 38 and a second membrane 40. The reticulated polyurethane foam pad 36 is impregnated with a deodorizing agent, such as activated carbon. The membranes 38, 40 and the reticulated polyurethane foam pad 36 may be secured together in any convenient way which integrates them into a single, unitary gas filter 34. By way of example, the membranes 38, 40 may be glued to the reticulated polyurethane foam pad 36.

In the prefered embodiment 10 of the invention, the first membrane 38 is treated to make it water repellent. The filter 34 is held in the housing 22 by means of a circular cap 42 which snaps onto the upper portion of the cylindrical wall 44 of the housing 22. The cap 42 includes a projection 46 which mates with a similar projection 48 on the upper surface of the wall 44. A lip 50 extends around the inner periphery of the cap 42. The lip 50 bears down upon the first membrane 38 thereby sealing the filter 34 in place within the housing 22 and insuring that any gases which enter the housing through the opening 28 must pass through the membrane 40, the foam pad 36, and the membrane 38. Accordingly, no gases can escape through the periphery 52 of the foam pad 36.

With continued reference to Figures 2—4, the central portion 54 of the base 30 of the housing 22 is somewhat thinner than the remainder of the base 30, thereby providing an air space 56 within the housing 22. The air space 56 surrounds the opening 28 in the housing 22.

Gas entering through the opening 28 passes through the gas filter 34 and is deodorized by the deodorizing agent impregnated into the reticulated polyurethane foam pad 36, activaed charcoal in the embodiment of the present invention.

The filter housing 22 can be made by injection molding any one of a variety of plastics. A material called "Nylon 6" which is supplied by Imperial Chemical Industries, of Great Britain is presently preferred, but other materials, including polyurethane or polypropylene, may be used.

As will be understood by those skilled in the art, while the gas filter and housing illustrated are substantially circular, other shapes, e.g. oval, square, or rectangular could equally well be employed. In addition, while one valuable use for the gas filter of the present invention is as a filter for an ostomy appliance, the invention may be applied to other uses. It has a number of advantageous features, which include its ability to be applied to a bag which has not been specially fabricated for use with a gas filter.

The membranes 38, 40 are microporous membranes, made of polyurethane.

The membranes 38, 40 may be a 0.1—0.15 mm thick polyurethane foil with controlled microporous holes of predetermined size therein, obtained by adding preground soluble material (e.g. rock salt) to a solution of polyurethane which is then extrusion calendered to thickness and width. When formed it is passed through hot water baths to dissolve the salts, leaving minute holes in the plastic foam.

A further alternative microporous membrane material is polyurethane or polyurethane into which is mixed a controlled pore size of chalk (calcium carbonate). This is then extruded leaving the chalk in situ which, because of its porosity, allows air to flow therethrough.

The foam pad 36 is made of a polyurethane foam. In particular, it is made of a reticulated polyurethane foam having calibrated pores.

The reticulated polyurethane foam pad 36 should be made of a material which has the characteristics indicated in the following Table A:

TABLE A

| | |
|---|---|
| Density | 27—30 kg/m³ |
| Compression resistance | 3.4—4.6 KPa |
| Ultimate elongation | 350% |
| Tensile strength | 200 KPa |
| Tear strength | 6 N/cm |
| Porosity | 65—85 (PPI) |

The water repellent layer which is applied to the first membrane 38 may be applied to the membrane 38 either by spraying, by immersion, or by deposition. The water repellent layer may be comprised of polytetrafluoroethylene (PTFE), fluorinated ethylene-propylene (FEP), silicone or wax. The coatings known by the Trade Names Scotchguard (3 ms) and Zepel may be used. The surface of the first membrane 38 to which the water repellent layer is applied may be pretreated to enhance the adhesion of the layer. Such pretreatment may be effected by a solvent, e.g., alcohol, methylethylketone, carbon tetrachloride, or acetone.

The pad is made of an open cell reticulated polyurethane foam carrying substantially 200 grams of carbon per square meter (e.g., 180 to 220 g/m²), and having an initial thickness of two millimeters, and a reduced thickness in the manufactured state of the filter of approximately 1 millimeter.

A satisfactory filter for ostomy bag use should have a gas permeability of at least about 8 ml per minutes per unit area at a pressure of 207 Pa (0.03 p.s.i.). The reticulated foam polyurethane material referred to above (Table A) has a gas permeability of about 30 ml per minutes per unit area when dry. Prior known filters stated by their manufacturers to be suitable for ostomy bag use have broadly comparable gas permeabilities when dry but become obstructed or clogged when wet. That is, their gas permeability when wet (i.e. after immersion in water) is negligible or zero and hence they are unsatisfactory. Gas filters in accordance with the previous particular description, however, exhibit a gas permeability of approximately 10 ml per minutes per unit area at 207 Pa (0.03 p.s.i.) even after being submerged in water at depths of up to 76 cm (30 inches) for periods up to 30 minutes. Moreover, filters according to the particular description herein retain a fully adequate deodorizing capability even after submersion as described above. This conclusion as to retention of deodorizing capability is based on the results of tests using air containing substantially 20 parts per million of $H_2S$ fed through the filter at approximately 30 ml/min.

The reason why the described arrangement is so surprisingly effective after water immersion is not fully understood, but it is believed that the water repellent layer forms a thin boundary film over the material defining the walls of the microporous holes, as well as over the top surface of the membrane 38. It is believed that this thin film prevents or greatly inhibits the access of water to the particles of activated carbon in the reticulated polyurethane foam pad.

**Claims**

1. A gas filter comprising a pad impregnated with activated carbon as a deodorizing agent and made of an open cell plastic foam material which is laminated together with a first microporous membrane, there being a layer of water repellent material on the surface of the membrane other than that to which said pad is attached, said layer being sufficiently thin that the pores in the membranes are not blocked against gas flow, characterised in that the pad is reticulated polyurethane foam having the characteristics indicated in the following Table A:

TABLE A

| | |
|---|---|
| Density | 27—30 kg/m³ |
| Compression resistance | 3.4—4.6 KPa |
| Ultimate elongation | 350% |
| Tensile strength | 200 KPa |
| Tear strength | 6 N/cm |
| Porosity | 65—85 (PPI); |

and further characterised in that the pad carries substantially 180 to 220 grams of carbon per square metre, and having a thickness in the manufactured state of the filter of approximately 1 millimetre.

2. A gas filter according to Claim 1 in which the water repellent layer is comprised of polytetrafluoroethlene (PTFE), fluorinated ethylene-propylene (FEP), silicone, or wax, and the surface of the first membrane to which said water repellent layer is applied is pretreated with a solvent selected from the group consisting of alcohol, methylethylketone, carbon tetrachloride, and acetone whereby the adhesion of the water repellent layer is enhanced.

3. A gas filter according to Claim 1 or 2 in which the first membrane is 0.1—0.15 mm thick polyurethane foil with controlled microporous holes of predetermined size therein, obtained by adding preground soluble material (e.g. rock salt) to a solution of polyurethane which is then extrusion calendered to thickness and width and then passed through hot water baths to dissolve said salt, thereby leaving minute holes.

4. A gas filter according to Claim 2 or 3 in which the microporous membrane is selected from the group consisting of PTFE and FEP.

5. A gas filter according to Claim 1 or 2 in which the first microporous membrane is polyurethane or polyurethane into which is mixed a controlled pore size of chalk (calcium carbonate) which remains *in situ* and being porous allows gas to flow therethrough.

6. A gas filter according to any one of Claims 1—5 in which the other side of the pad is laminated to a second microporous membrane which is of substantially the same material as the first.

7. A gas filter according to any preceding claim further comprising a filter housing for containing said gas filter, said filter housing being comprised of a plastic housing having a base wall with a hole formed therethrough for entry of gas to be deodorized and a peripheral wall of which traps said filter pad within said housing.

**Patentansprüche**

1. Gasfilter mit einem Kissen, das mit aktivierter Kohle als deodorisierendes Mittel imprägniert ist und aus einem offenzelligen Kunststoffschaummaterial hergestellt ist, das zusammen mit einer ersten mikroporösen Membran laminiert ist, wobei sich eine Lage aus wasserabstoßendem Material auf der Fläche der Membran befindet, die nicht die ist, auf der das Kissen aufgebracht ist, wobei die Lage ausreichend dünn ist, daß die Poren in der Membran nicht gegen Gasfluß blokkiert sind, dadurch gekennzeichnet, daß das Kissen ein netzförmiger Polyurethanschaum mit den in der folgenden Tabelle A angegebenen Eigenschaften ist:

TABELLE A

| | |
|---|---|
| Dichte | 27—30 kg/m³ |
| Druckwiderstand | 3,4—4,6 KPa |
| maximale Verlängerung | 350% |
| Zugfestigkeit | 200 KPa |
| Reißfestigkeit | 6 M/cm |
| Porosität | 65—85 (PPI), |

und ferner dadurch gekennzeichnet ist, daß das Kissen im wesentlichen 180 bis 220 g Kohlenstoff

pro m² trägt und eine Dicke im hergsetellten Zustand des Filters von ungefähr 1 mm aufweist.

2. Gasfilter nach Anspruch 1, wobei die wasserabstoßende Lage aus Polytetrafluorethylen (PTFE), fluoriniertem Ethylen-Propylen (FEP), Silikon oder Wachs besteht, und die Fläche der ersten Membran, auf der die wasserabstoßende Lage aufgebracht wird, mit einem Lösungsmittel vorbehandelt wird, das ausgewählt wird aus der Gruppe, bestehend aus Akohol, Methylethylketon, Kohlenstofftetrachlorid und Aceton, wodurch die Adhäsion der wasserabstoßenden Lage erhöht wird.

3. Gasfilter nach Anspruch 1 oder 2, wobei die erste Membran eine 0,1 bis 0,15 mm dicke Polyurethanfolie mit gesteuerten mikroporösen Löchern bestimmter Größe ist, die erhalten wird durch Hinzufügen von vorgemahlenen lösbaren Materialien (z.B. Steinsalz) zu einer Lösung aus Polyurethan, die dann zu einer Dicke und Breite extrusionskalandert wird und dann zum Lösen des Salzks durch heiße Wasserbäder geleitet wird, wodurch kleine Löcher zurückbleiben.

4. Gasfilter nach Anspruch 2 oder 3, wobei die mikroporöse Membran ausgewählt wird aus der Gruppe bestehend aus PTFE und FEP.

5. Gasfilter nach Anspruch 1 oder 2, wobei die erste mikroporöse Membrane Polyethylen oder Polyurethan ist, in das Kalk mit einer gesteuerten Porengröße (Calciumcarbonat) gemischt wird, der in situ zurückbleibt und porös ist, wodurch Gas hindurchfließen kann.

6. Gasfilter nach einem der Ansprüche 1 bis 5, wobei die andere Seite des Kissens mit einer zweiten mikroporösen Membran laminiert ist, die aus im wesentlichen dem gleichen Material wie die erste besteht.

7. Gasfilter nach einem der vorangehenden Ansprüche, ferner mit einem Filtergehäuse zum Aufnehmen des Gasfilters, wobei das Filtergehäuse aus einem Plastikgehäuse mit einer Basiswand, in der ein Loch zum Eintritt von zu deodorisierendem Gas ausgebildet ist, und einer äußeren Wand, mit der das Filterkissen innerhalb des Gehäuses gehalten wird.

**Revendications**

1. Filtre à gaz comprenant un tampon imprégné de charbon actif en guise d'agent désodorisant et fait d'une mousse de matière plastique à cellules ouvertes qui forme un stratifié avec une première membrane microporeuse, une couche de matière hydrofuge étant prévue sur la surface de la membrane autre que celle à laquelle ledit tampon est fixé, ladite couche étant assez mince pour que les pores de la membrane n'empêchent pas l'écoulement de gaz, caractérisé en ce que le tampon est une mousse de polyuréthane réticulée ayant les caractéristiques indiquées dans le Tableau A ci-dessous:

TABLEAU A

| | |
|---|---|
| Masse volumique | 27—30 kg/cm³ |
| Résistance à la compression | 3,4—4,6 kPa |
| Allongement à la rupture | 350% |
| Résistance à la traction | 200 kPa |
| Résistance à la déchirure | 6 N/cm |
| Porosite | 65—85 (PPI) |

et caractérisé en outre en ce que le tampon porte sensiblement 180 à 220 grammes de carbone par mètre carré, et ayant une épaisseur, dans l'état fabriqué du filtre, d'environ 1 millimètre.

2. Filtre à gaz selon la revendication 1, dans lequel la couche hydrofuge se compose de polytétrafluoroéthylène (PTFE), d'éthylène-propylène fluoré (FEP), de silicone, ou de cire, et la surface de la première membrane à laquelle ladite couche hydrofuge est appliquée est traitée au préalable par un solvant choisi entre l'alcool, la méthyléthylcétone, le tétrachlorure de carbone et l'acétone, pour que l'adhérence de la couche hydrofuge soit accrue.

3. Filtre à gaz selon la revendication 1 ou 2, dans lequel la première membrane est une feuille de polyuréthane épaisse de 0,1 à 0,15 mm percée de trous microporeux contrôlés de taille prédéterminée, obtenue en ajoutant une matière soluble broyée au préalable (sel gemme par exemple) à une solution de polyuréthane, que l'on lamine ensuite par extrusion à l'épaisseur et à la largeur volues, puis qu'on fait passer dans des bains d'eau très chaude pour dissoudre ledit sel, ce qui laisse subsister de tout petits trous.

4. Filtre à gaz selon la revendication 2 ou 3, dans lequel la membrane microporeuse se compose de PTFE ou de FEP.

5. Filtre à gaz selon la revendication 1 ou 2, dans lequel la première membrane microporeuse est faite de polyéthylène ou de polyuréthane auquel est mélangée de la craie (carbonate de calcium) à porosimétrie contrôlée, qui reste *in situ* et qui, étant poreuse, permet aux gaz de passer au travers.

6. Filtre à gaz selon l'une quelconque des revendications 1 à 5 dans lequel l'autre face du tampon forme un stratifié avec une seconde membrane microporeuse qui est faite sensiblement de la même matière que la première.

7. Filtre à gaz selon une quelconque des revendications précédentes, comprenant en outre un logement de filtre destiné à contenir ledit filtre à gaz, ledit logement de filtre étant constitué d'un logement en matière plastique dont la paroi de base est percée d'un trou pour permettre l'entrée du gaz à désodoriser, et qui comporte une paroi périphérique qui emprisonne ledit tampon filtrant à l'intérieur dudit logement.

_Fig_ _3_

_Fig_ _4_

_Fig_ _1_

_Fig_ _2_